# EUROPEAN PATENT SPECIFICATION

(11) **EP 3 492 067 B1**
(45) Date of publication and mention of the grant of the patent: **19.10.2022**
(21) Application number: 17834529.4
(22) Date of filing: 28.07.2017
(51) Int. Cl.: A61K 8/60, A61K 8/34, A61K 31/05, A61K 31/7034, A61P 17/14, A61P 43/00, A61Q 7/00, C07H 15/203

(54) **TYPE-17 COLLAGEN PRODUCTION PROMOTER**
TYP-17-KOLLAGEN-PRODUKTIONSPROMOTER
PROMOTEUR DE PRODUCTION DE COLLAGÈNE.

(30) Priority: 28.07.2016 JP 2016148355; 16.05.2017 JP 2017097329
(43) Date of publication of application: 05.06.2019
(73) Proprietor: Tomoike Bio Ltd., Shatin, N.T. (HK)
(72) Inventor: HAMADA, Hiroki, Okayama-shi Okayama 701-1205 (JP); HOSODA, Ryusuke, Sapporo-shi Hokkaido 060-0042 (JP); ONO, Tsubasa, Okayama-shi Okayama 703-8203 (JP)
(74) Representative: Wynne-Jones IP Limited
(86) International application number: PCT/JP2017/027437
(87) International publication number: WO 2018/021527

(56) References cited:
- EP-A1- 2 832 344
- WO-A1-2016/102177
- WO-A1-2017/080958
- WO-A1-2018/034328
- WO-A2-2014/095289
- JP-A- H11 322 577
- JP-A- 2001 072 552
- JP-A- 2008 239 576
- JP-A- 2009 161 509
- JP-A- 2010 030 911
- JP-A- 2013 523 883
- JP-A- 2014 171 436
- JP-A- 2016 028 018
- KR-B1- 101 741 808
- US-A1- 2015 098 987
- DAISUKE SATO ET AL: "Synthesis of glycosides of resveratrol, pterostilbene, and piceatannol, and their anti-oxidant, anti-allergic, and neuroprotective activities", BIOSCIENCE, BIOTECHNOLOGY AND BIOCHEMISTRY, vol. 78, no. 7, 9 June 2014 (2014-06-09), pages 1123-1128, XP055470270, JP ISSN: 0916-8451, DOI: 10.1080/09168451.2014.921551

## Description

### Technical Field

The present invention relates to a use of a composition for promoting Type-17 collagen production.

### Background Art

It has been known that the deficiency of Type-17 collagen present around the hair follicle cells may deteriorate head hair, thereby causing generation of gray hair, hair loss, and the like (Non-patent Literature 1). Type-17 collagen, which is also called, for example, BP180, is a protein having a membrane-spanning domain. In an organism, this protein is known to be involved in a function of enabling epithelial tissues to be adhered to the basement membrane. It is also known that the deficiency of this protein develops bullous pemphigoid (Patent Document 1).

Minoxidil has been known as an active ingredient of a hair-growing agent. However, minoxidil is a compound originally developed as an active ingredient of an antihypertensive. Therefore, there has been a concern regarding serious side effects of an internal agent containing minoxidil. For example, some fatal cases have been reported.

### Citation List

### Patent Literature

Patent Literature 1: JP2007-129938A
Patent Literature 2: WO2014095289 A2
Patent Literature 3: WO2016102177 A1
Patent Literature 4: EP2832344 A1
Patent Literature 5: JP2014-171436 A

### Non-Patent Literature

Non-Patent Literature 1: Science, (2016) Vol. 351, Issue 6273, p. 575
Non-Patent Literature 2: Daisuke Sato et al.; Bioscience, biotechnology and biochemistry, (2014), Vol. 78, No. 7, p.1123-1128

### Summary of Invention

### Technical Problem

There has been an increased demand for various compositions capable of preventing deterioration of body hair typified by head hair (e.g., gray hair, hair loss) due to aging or the like, such as food, cosmetics, pharmaceutical products, and the like. Moreover, to satisfy the market, it is necessary to guarantee a certain degree of safety of such compositions.

Non-patent Literature 1 merely discloses that the deficiency of Type-17 collagen causes deterioration of hair, and nowhere discloses a means for supplying Type-17 collagen to an organism, a method for retaining Type-17 collagen in an organism, or the like.

Non-patent literature 2 discloses synthesis of glycosides of resveratrol, pterostilbene, and piceatannol, and their antioxidant, anti-allergic, and neuroprotective activities.

Patent Literature 2 discloses a method of treating hair ageing through application to hair fibres of a composition comprising a nuclear factor erythroid-2 related factor 2 agonist.

Patent Literature 3 discloses an oral or topical composition comprising a nuclear factor erythroid-2 related factor 2 agonist and a liver X receptor agonist.

Patent Literature 4 discloses a composition containing carotenoid and pterostilbene and an external preparation for skin or a functional food containing the composition.

Patent Literature 5 discloses a production method of a regioselectively and stereoselectively glycosidated resveratrol glycoside.

An object of the present invention is to provide a method for supplying or retaining Type-17 collagen to/in an organism, as well as a composition for use in the method. Solution to Problem

The inventors of the present invention carried out extensive research, and found that at least one compound selected from the group consisting of stilbene compounds and glycosides thereof is capable of promoting Type-17 collagen production in an organism.

The present invention was accomplished based on the above findings. The invention is defined by the appended claims.

Item 1. According to an aspect of the invention there is provided a cosmetic use for promoting Type-17 collagen production of a cosmetic composition comprising pterostilbene or a glycoside thereof as an active ingredient.

Item 2. The use according to Item 1, wherein the glycoside is a glycoside in which pterostilbene and the hydroxyl group at position 1 of a saccharide are linked by a glycosidic linkage.

Item 3. The use according to any one of Items 1 or 2, wherein the glycoside is a glycoside in which pterostilbene and a monosaccharide are linked by a glycosidic linkage.

Item 4. The use according to Item 3, wherein the monosaccharide is at least one member selected from the group consisting of glucose, threose, lyxose, allose, psicose, sedoheptulose, and volemitol.

Item 5. The use according to any one of Items 1 or 2, wherein the glycoside is a glycoside in which pterostilbene and a polysaccharide constituted of two or more monosaccharides are linked by a glycosidic linkage.

Item 6. The use according to Item 5, wherein the saccharide residue of the polysaccharide linked to the pterostilbene by a glycosidic linkage is a glucose residue.

Item 7 (not claimed). A composition for external use, comprising the Type-17 collagen production promoter according to any one of Items 1 to 6.

Item 8 (not claimed). A method for producing the composition for external use according to Item 7, comprising the step of mixing the stilbene compound and/or a glycoside thereof with one or more components acceptable for a composition for external use.

Item 9 (not claimed). A composition for internal use, comprising the Type-17 collagen production promoter according to any one of Items 1 to 6.

Item 10 (not claimed). A hair-growing agent, comprising the Type-17 collagen production promoter according to any one of Items 1 to 7 or 9.

Item 11 (not claimed). Use of the Type-17 collagen production promoter according to any one of Items 1 to 7 or 9, for the production of a hair-growing agent.

Item 12 (not claimed). A hair-growing method, comprising the step of applying the Type-17 collagen production promoter according to any one of Items 1 to 7 or 9 to the skin or scalp of a human who desires hair growth.

Item 13 (not claimed). In some examples, the pterostilbene compound can be represented by some examples of formula (2) below: 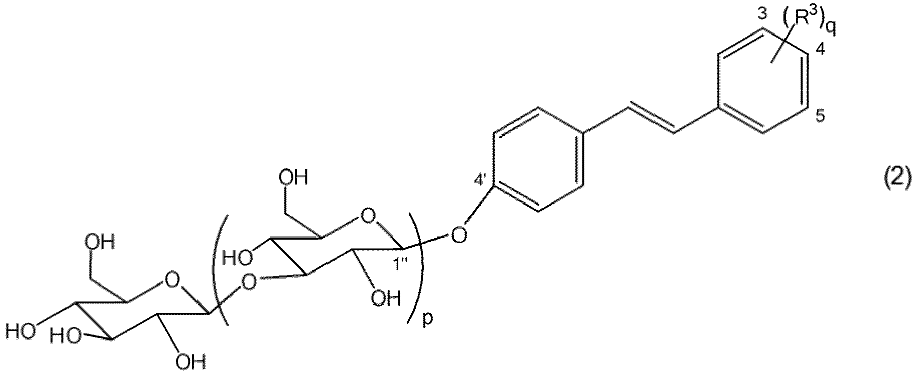 wherein R³ represents a C₁₋₄ alkoxy group; p represents an integer of 0 to 15; and q represents an integer of 1 to 5. However, the invention itself is limited as set out in the claims.

Item 14 (not claimed). The example compound according to Item 13, wherein in formula (2), R³ is selected from a methoxy group, ethoxy group, n-propoxy group, isopropoxy group, n-butoxy group, and t-butoxy group.

Item 15 (not claimed). The example compound according to Item 13 or 14, wherein in formula (2), q is 2.

Item 16 (not claimed). The example compound according to any one of Items 13 to 15, wherein in formula (2), the group R³ is coordinated to at least one of position 3, position 4, and position 5.

Item 17 (not claimed). The example compound according to any one of Items 15 to 18, wherein in formula (2), the oxygen atom positioned between the stilbene skeleton and the glucopyranose ring is linked to position 1" of the glucopyranose ring by a β linkage.

Item 18 (not claimed). The example compound according to any one of Items 13 to 17, wherein in formula (2), when p is an integer of 1 or more, all of the (1-4) linkages between glucopyranose rings are α linkages.

Item 19 (not claimed). The example compound according to any one of Items 13 to 18, wherein p in the formula is 2 or more.

### Advantageous Effects of Invention

The present invention provides a composition for use in the promotion of Type-17 collagen production (Type-17 collagen production promoter). As the preferable form of the composition of the present invention, the composition of the present invention may be in the form of a composition for external use, or a composition for internal use. The Type-17 collagen production promoter of the present invention may be in the form of a hair-growing agent, as a more preferable form.

Further, the pterostilbene and a glycoside thereof as an active ingredient of the Type-17 collagen production promoter of the present invention are compounds contained in an edible plant. Thus, the compound is highly safe for humans, and has minor side effects.

### Brief Description of Drawings

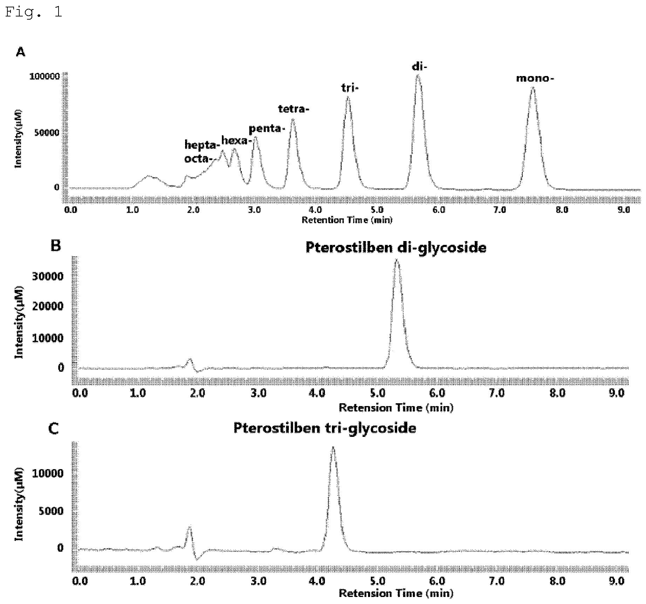

Fig. 1 shows an HPLC chart for confirming production of the pterostilbene polyglycoside shown in Production Example 2. In each graph in the figure, the vertical axis denotes UV intensity (µV), and the horizontal axis denotes retention time (min). In graph A, mono- represents a monoglycoside, di- represents a diglycoside, tri- represents a triglycoside, tetra- represents a tetraglycoside, penta- represents a pentaglycoside, hexa-represents a hexaglycoside, hepta- represents a heptaglycoside, and octa- represents an octaglycoside. Graph B shows an isolated pterostilbene diglycoside. Graph C shows an isolated pterostilbene triglycoside.

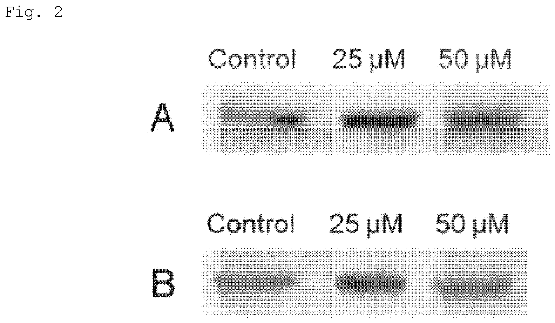

Fig. 2 shows results of a test for confirming the expression amount of Type-17 collagen using the pterostilbene and pterostilbene monoglycoside shown in Example 2. Graph A in the figure shows the results of western blotting showing the expression amount of Type-17 collagen protein in a human skin model after pterostilbene was added, and graph B shows the results of addition of pterostilbene monoglycoside.

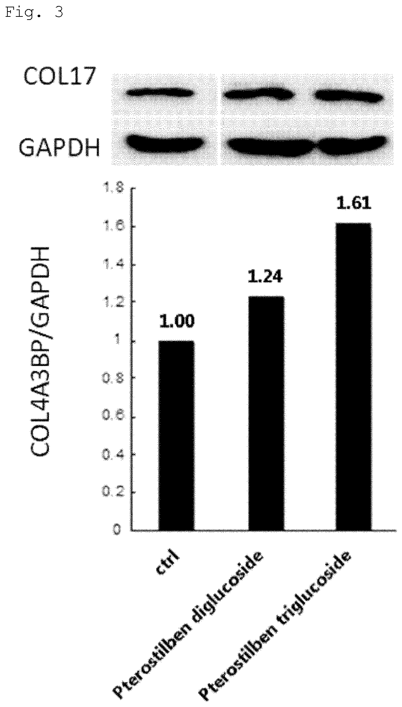

Fig. 3 shows results of a test for confirming the amount of Type-17 collagen using the pterostilbene polyglycoside (pterostilbene diglycoside and pterostilbene triglycoside) shown in Example 3.

### Description of Embodiments

### Type-17 Collagen Production Promotor

The Type-17 collagen production promoter of the present invention comprises, as an active ingredient, at least one compound selected from the group consisting of pterostilbene and glycosides thereof.

The stilbene compound mentioned above is a compound having a stilbene skeleton. An example is a compound represented by formula (1) below.

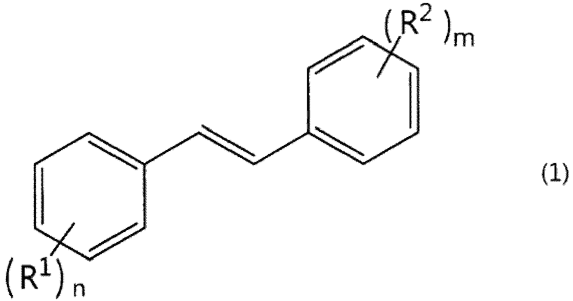

In formula (1) shown above, R¹ and R² are the same or different, and each represents a hydroxyl group or alkoxy group.

In formula (1) shown above, n is an integer of 0 to 5. n is preferably an integer of 0 to 4, more preferably an integer of 0 to 3, further preferably an integer of 1 to 2.

In formula (1) shown above, m is an integer of 0 to 5. m is preferably an integer of 0 to 4, more preferably an integer of 0 to 3, further preferably an integer of 1 to 2.

The sum of n and m is not less than 1. m is preferably not less than 1 and not more than 5, more preferably not less than 1 and not more than 4, further preferably not less than 2 and not more than 4.

The positions of the benzene ring to which R¹ and R² are coordinated are not particularly limited. For example, R¹ and R² may be coordinated to any one of position 2, position 3, position 4, position 5, position 6, position 2', position 3', position 4', position 5', and position 6' of the benzene ring of the compound represented by formula (1). Among these, R¹ and R² are preferably coordinated to at least one of position 3, position 4, position 5, position 3', position 4', and position 5' .

The positions of the benzene ring to which R¹ and R² are coordinated are not restricted by the positions to which they are coordinated. They may be arbitrarily coordinated to any of the above positions.

"Alkoxy group" refers to a group having a structure in which a hydrocarbon group is linked to oxygen atom, and is not particularly limited insofar as it has this structure. Examples include methoxy group, ethoxy group, n-propoxy group, isopropoxy group, n-butoxy group, and t-butoxy group in which the hydrocarbon group is a C₁₋₄ alkyl group; phenoxy group, and 1-naphthyloxy group in which the hydrocarbon group is an aryl group; allyloxy group in which the hydrocarbon group is an alkenyl group; cyclohexyloxy group in which the hydrocarbon group is a cycloalkyl group; benzyloxy group in which the hydrocarbon group is a cycloalkyl group; and the like. Among these, methoxy group or ethoxy group is preferable, and methoxy group is most preferable.

Specific examples of the stilbene compounds (not claimed) include resveratrol, piceatannol, rhapontigenin, oxyresveratrol, gnetol, pinostilbene, and like compounds. Among these compounds, resveratrol, piceatannol, or pinostilbene are preferable. The stilbene compounds according to the claimed invention are pterostilbene and glycosides thereof.

The glycoside of pterostilbene compound as an active ingredient of the Type-17 collagen production promoter of the present invention mentioned above is not particularly limited.

As one embodiment of the glycoside described above, a glycoside in which the stilbene compound described above is linked to a monosaccharide by a glycosidic linkage (this glycoside may hereinafter be referred to as a "monoglycoside" in this specification) may be exemplified.

The glycosidic linkage described above is not particularly limited. Examples of the glycosidic linkage include an embodiment in which the hydroxyl group of a monosaccharide is linked to the hydroxyl group of the stilbene compound described above by a glycosidic linkage. An embodiment in which the hydroxyl group at position 1 of a monosaccharide is linked to an arbitrary hydroxyl group of the stilbene compound by a glycosidic linkage is preferable.

Further, the steric direction of the glycosidic linkage described above is not particularly limited, and may be any of α-type and β-type. In view of glycoside stability in an organism, β-type is preferable.

The monosaccharide described above is not particularly limited. Examples of the monosaccharide include glucose, threose, lyxose, allose, psicose, sedoheptulose, volemitol, and like compounds. Among these compounds, glucose is preferable.

As another embodiment of the glycoside described above, a glycoside in which a stilbene compound is linked to a polysaccharide constituted of two or more monosaccharides by a glycosidic linkage (this glycoside may hereinafter be referred to as a "polyglycoside" in this specification) may be exemplified.

The monosaccharides constituting the polysaccharide described above are not particularly limited. Examples of the monosaccharides include glucose, threose, lyxose, allose, psicose, sedoheptulose, volemitol, and the like. Among these compounds, glucose is preferable.

The number of monosaccharides contained in the polysaccharide described above is not particularly limited, insofar as two or more monosaccharides are contained. For example, 3, 4, 5, 6, 7, 8, 9, 10, 11, 12, 13, 14, or 15 monosaccharides may be contained.

The form of the linkage between the monosaccharides in these polysaccharides are not particularly limited, and is generally a glycosidic linkage.

The coordination form in the glycosidic linkage described above is not particularly limited. More specifically, examples of the form include 1-4 linkage, 1-6 linkage, 1-3 linkage, and the like. Further, the steric direction of the glycosidic linkage is also not particularly limited, and may be any of α linkage and β linkage.

The steric direction in the glycosidic linkage between the stilbene compound described above with a polysaccharide constituted of two or more monosaccharides in a polyglycoside is not particularly limited. Examples include α linkage and β linkage. In view of glycoside stability in an organism, β-type is preferable.

The amount of the at least one compound (may hereinafter be referred to as an "active ingredient") selected from the group consisting of pterostilbene and glycosides thereof contained in the Type-17 collagen production promoter of the present invention is not particularly limited, insofar as the effects of the present invention can be exerted. For example, the amount of the active ingredient per 100 mass% of the Type-17 collagen production promoter is generally about 0.000001 mass% to 100 mass%.

Both of the stilbene compound and the glycoside thereof described above may be known substances, or may easily be produced from a known substance by a known method. Further, the glycoside of the stilbene compound may be produced by a method of glycosidation of a stilbene compound through Koenigs-Knorr glycosidation, which is an organic synthesis method, a method using a glycosyltransferase, or the like.

The polyglycoside may be produced from the monoglycoside described above through an organic synthesis method, or by using a glycosyltransferase.

Further, the stilbene compound and a glycoside thereof may be obtained from a plant by using a known extraction method.

As shown in the Examples below, the Type-17 collagen production promoter of the present invention exerts a Type-17 collagen production promoting effect by being applied to epidermis. As described above, the deficiency of Type-17 collagen may deteriorate head hair or like body hair, thereby causing generation of gray hair, hair loss, and the like. Therefore, the effects of preventing deterioration of head hair, eyebrows, eyelashes, beard, or like body hair (prevention of gray hair or hair loss), and the effects of promoting growth, generation, and the like of these body hairs can be expected by internal or external use of the Type-17 collagen production promoter of the present invention.

Therefore, the Type-17 collagen production promoter of the present invention may be used as an agent for external use, and as an agent for internal use (hereinafter, these agents may be referred to as a "composition for external use" and a "composition for internal use," respectively).

### Composition for External Use

The composition for external use of the present invention comprises the Type-17 collagen production promoter described above.

The amount of the at least one compound selected from the group consisting of pterostilbene and glycosides thereof contained in the composition for external use is not particularly limited insofar as the effects of the present invention can be exerted. For example, the amount of the active ingredient per 100 mass% of the composition for external use is generally about 0.000001 mass% to 100 mass%.

The subjects to which the composition for external use of the present invention is applied are not particularly limited, and include any subjects who expect the above effects of the Type-17 collagen production promoter; more specifically, any subjects who wish to alleviate deterioration of head hair, eyebrows, eyelashes, beard, or like body hair (gray hair, hair loss, etc.), and any subjects who desire the effects of promoting growth, generation, and the like of these body hairs.

The amount to be used of the composition for external use of the present invention is not particularly limited, insofar as the effects described above can be exerted. Specifically, the amount may be determined in accordance with the amount for a cosmetic composition described below, which is a form of the composition for external use of the present invention. More specifically, the active ingredient of the composition for external use may generally be used in an amount of about 1 mg to 5 mg per 1 cm² of the skin or scalp.

The form of the composition for external use of the present invention is not particularly limited. Examples include cosmetic compositions (including quasi-drugs such as medicinal cosmetics) and pharmaceutical compositions (mainly external preparations such as aerosols, liquids, extracts, percutaneous absorbent, suspensions, emulsions, spirits, patches, tinctures, cataplasms, aromatic water, liniments, fluid extracts, lotions, and the like).

The compositions for external use described above may be applied to any parts in need of the effects obtained from the compositions. More specifically, if the user expects the effects of alleviating the deterioration of head hair (gray hair, hair loss, or the like), the composition for external use is applied to the scalp; if the user expects the effects of alleviating the deterioration of eyebrows, the composition for external use is applied to the eyebrows.

The frequency in use of the composition for external use described above is not particularly limited. For example, the frequency in use may be appropriately set according to the age, sex, or the like of the subject, or the desired extent of the effects described above.

### Method for Producing Composition for External Use

The method for producing the composition for external use of the present invention described above comprises a step of mixing at least one compound selected from the group consisting of stilbene compounds and glycosides thereof with one or more components that can be added to a composition for external use.

The at least one compound selected from the group consisting of pterostilbene and glycosides thereof is as defined above.

The components that can be added to the composition for external use is not particularly limited. Examples include components that can be added to a pharmaceutical composition or a cosmetic composition exemplified above as a form of the composition for external use.

### Cosmetic Composition

As mentioned above, the composition for external use of the present invention may be in the form of a cosmetic composition.

The amount of the at least one compound selected from the group consisting of pterostilbene and glycosides thereof contained in the cosmetic composition is not particularly limited insofar as the effects of the present invention can be exerted. For example, the amount of the active ingredient per 100 mass% of the composition is generally about 0.000001 mass% to 100 mass%.

The form of the cosmetic composition described above is not particularly limited. Examples of the form include lotions (liquids), mousse, gels, jellies, emulsions, suspensions, creams, ointments, sheets, aerosols, sprays, and the like. Having these forms, the cosmetic composition may be applied not only to skin, but also to scalp.

The form of the cosmetic composition is not particularly limited. Examples of the form include mascara, eyebrow powder, eye shadow, eyeliner, and like make-up cosmetics; cosmetic water, emulsions, skin creams, lotions, oils, face masks, and like skin-care cosmetics; facial cleanser, makeup remover, body soap, and like skin cleansers; shampoo, rinse, conditioners, hair-styling agents, hair-growing agents, and like hair cosmetics; massage agents; cleaning agents; and the like.

The application amount of the cosmetic composition described above may be appropriately set according to the sex, age, or the like of the subject; or the application form, desired extent of the effects, and the like of the cosmetic composition. For example, the active ingredient of the cosmetic composition may be used generally in an amount of about 1 mg to 5 mg per 1 cm² of the skin or scalp.

The cosmetic composition described above may suitably be used, for example, for a hair-growing agent or the like based on the effects of the Type-17 collagen production promoter. The "hair growth" in the present invention may also mean, for example, hair generation, hair growth, prevention of hair loss, and the like.

### Composition for Internal Use

The composition for internal use of the present invention comprises the Type-17 collagen production promoter described above.

The amount of the at least one compound selected from the group consisting of pterostilbene and glycosides thereof contained in the composition for internal use is not particularly limited, insofar as the effects of the present invention can be exerted. For example, the amount of the active ingredient per 100 mass% of the composition for internal use is generally about 0.000001 mass% to 100 mass%.

The subjects to which the composition for internal use of the present invention is applied are not particularly limited, and include any subjects who expect the above effects of the Type-17 collagen production promoter, more specifically, any subjects who wish to alleviate deterioration of head hair, eyebrows, eyelashes, beard, or like body hair (gray hair, hair loss, etc.); and any subjects who desire the effects of promoting growth, generation, and the like of these body hairs.

The amount of the composition for internal use of the present invention is not particularly limited insofar as the effects described above can be exerted. For example, the amount may be determined in accordance with the amount for a food or beverage composition described below, which is a form of the composition for internal use of the present invention. For example, the intake for a human adult (having a weight of, for example, 60 kg) per day is generally about 5 mg to 10 mg, in terms of the amount of active ingredient of the composition for internal use.

The form of the composition for internal use of the present invention is not particularly limited. Examples include food or beverage compositions, patient food, pharmaceutical compositions (mainly including internal agents, such as liquids, extracts, elixirs, capsules, granules, pills, suspensions, milky agents, suppositories, powdered drug, spirits, tablets, syrups, infusions, decoctions, tinctures, eye-drops, lozenges, ointments, aromatic water, limonades, fluidextracts, or the like), and the like.

Among these forms, food or beverage compositions are preferable.

### Method for Producing Composition for Internal Use

The method for producing the composition for internal use of the present invention comprises a step of mixing at least one compound selected from the group consisting of stilbene compounds and glycosides thereof with one or more components that can be added to a composition for internal use.

At least one compound selected from the group consisting of pterostilbene and glycosides thereof is as defined above.

The components that can be added to the composition for internal use are not particularly limited. Examples include components that can be added to a pharmaceutical composition or a food or beverage composition exemplified above as a form of the composition for internal use, or other components based on these components. For example, the intake for a human adult (having a weight of, for example, 60 kg) per day is generally about 5 mg to 10 mg, in terms of the amount of active ingredient of the composition for internal use.

### Food or Beverage Composition

As mentioned above, the composition for internal use of the present invention may be in the form of a food or beverage composition.

The amount of the at least one compound selected from the group consisting of pterostilbene and glycosides thereof contained in the food or beverage composition of the present invention is not particularly limited, insofar as the effects of the present invention can be exerted. For example, the amount of the active ingredient per 100 mass% of the composition is generally about 0.000001 mass% to 100 mass%.

The food or beverage composition of the present invention may encompass any of food for specified health use, functional display food, food with nutrient function claims, and like food with health claims (including beverages); dietary supplements, health supplements, and nutritionally modulated food, etc.; as well as general food (including beverages).

It is sufficient that these food or beverage compositions are in the form of conventional food or beverage. For example, in addition to the general food or beverage forms (obvious food), the compositions are preferably food for specified health use or functional display food exhibiting and ensuring, as the functions thereof, effects of alleviating deterioration of head hair, eyebrows, eyelashes, beard, or like body hair (gray hair, hair loss, etc.). The food or beverage compositions described above may claim the purpose of maintaining liveliness, health, beauty, preventive medicine, daily health, fatigue recovery, skin care, health, skin health maintenance, as well as scalp care. However, the invention itself is limited to use of a cosmetic composition, as defined in the claims.

The form of the food or beverage composition described above is not particularly limited. Specific examples of the food or beverage compositions include drinks, such as soft drinks, carbonated drinks, energy drinks, fruit drinks, and lactic-acid drinks; frozen desserts, such as ice cream and shaved ice; confectionery, such as gum, chocolate, candies, tablet candies, snacks, jellies, jams, creams, and gummy candies; noodles, such as *soba, udon,* instant noodles, and Chinese noodles; fishery and livestock processed foods, such as *kamaboko* [fish sausage], ham, and sausage; dairy products, such as processed milk and fermented milk; fats and oils, and fat-and-oil processed foods, such as salad oil, mayonnaise, whipped cream, and dressing; seasonings, such as marinade sauce and basting sauce; and soup, salad, side dishes, pickles, bread, cereal, and the like.

For example, the food or beverage composition of the present invention may be in a preparation form generally provided as a supplement, including a solid preparation such as powder, granules, capsules, lozenges, or tablets; liquid preparations such as syrups or drinks; and the like.

The intake amount of the food or beverage composition described above may be appropriately set according to the sex, age, or the like of the subject; or the application form, desired extent of the effects, and the like of the food or beverage composition. For example, the intake for a human adult (having a weight of, for example, 60 kg) per day is generally about 5 mg to 10 mg, in terms of the amount of active ingredient of the food or beverage composition.

### Pterostilbene Compound

The pterostilbene compound of the present invention is represented by formula (2) below. 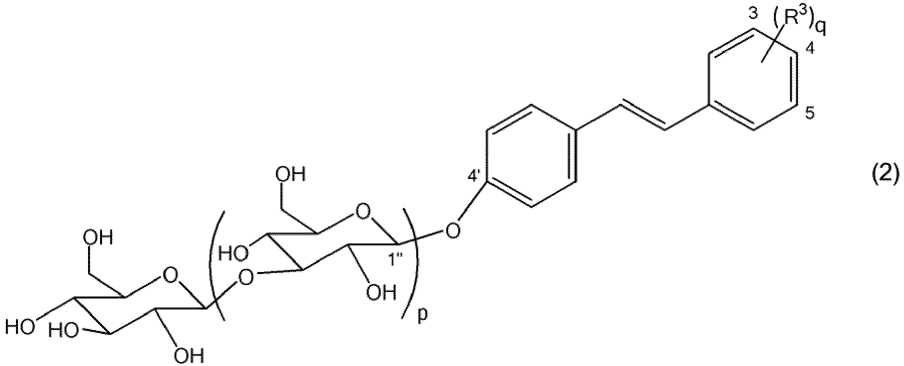 wherein R³ in formula (2) represents a C₁₋₄ alkoxy group. The "alkoxy group" refers to a group having a structure in which a C₁₋₄ alkyl group is linked to oxygen atom, and is not particularly limited insofar as it has this structure. Examples include a methoxy group, ethoxy group, n-propoxy group, isopropoxy group, n-butoxy group, t-butoxy group, and the like. Among these, methoxy group or ethoxy group is preferable, and methoxy group is most preferable.

In formula (2) above, p is an integer of 0 to 15. Among these, in light of the water solubility of pterostilbene compound, p is preferably 2 or more. Further, in light of the production cost or the like, p is preferably 2.

In formula (2) above, q is an integer of 1 to 5. Among these, q is preferably 2.

In formula (2) above, the position where the group R³ is substituted is not particularly limited. Examples of the position include at least one of position 2, position 3, position 4, position 5, and position 6. The position where the group R³ is substituted is preferably at least one of position 3, position 4, and position 5. The group R³ is most preferably substituted at position 3 and position 5. Therefore, in formula (2) above, a compound in which methoxy groups are substituted at position 3 and position 5 is most preferable.

In formula (2) above, the linkage state of the oxygen atom positioned between the stilbene skeleton and the glucopyranose ring with position 1" of the glucopyranose ring is not particularly limited. For example, the linkage of the oxygen atom positioned between the stilbene skeleton and the glucopyranose ring with position 1" of the glucopyranose ring may be an α linkage or a β linkage. The linkage is preferably a β linkage. The oxygen atom positioned between the stilbene skeleton and the glucopyranose ring herein means an oxygen atom linked to position 4' of the stilbene skeleton.

In formula (2) above, when p is an integer of 1 or more, a (1-4) linkage is present between the glucopyranosides in the pterostilbene compound. The type of the (1-4) linkage described above is not particularly limited. For example, the linkage may be an α linkage or a β linkage. In light of ease of production, the linkage is preferably an α linkage. More preferably, all linkages are α linkages.

The pterostilbene compound described above may be used as an active ingredient of the Type-17 collagen production promoter disclosed in the present specification.

### Method for Producing Pterostilbene Compound

The method for producing the pterostilbene compound described above is not particularly limited. Examples include the following methods. A different production method may be used depending on whether P is 0 or an integer of 1 or more in formula (2) above.

### The case where p is 0

An example of the method for producing a pterostilbene compound in which p is 0 is shown below. The pterostilbene compound in which p is 0 is a compound represented by formula (3) below. 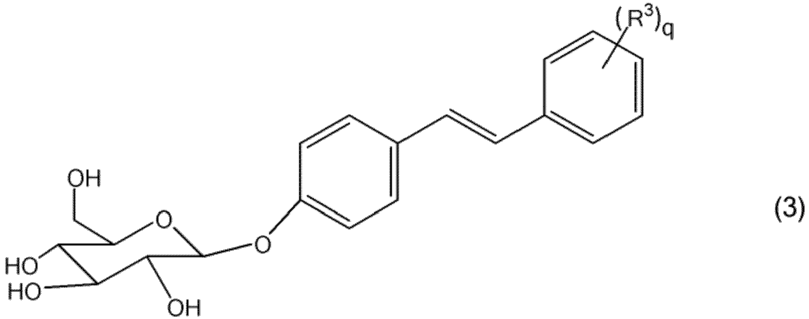 wherein R³ represents a C₁₋₄ alkoxy group; and q represents an integer of 1 to 5.

The compound represented by formula (4) is prepared as a starting material. 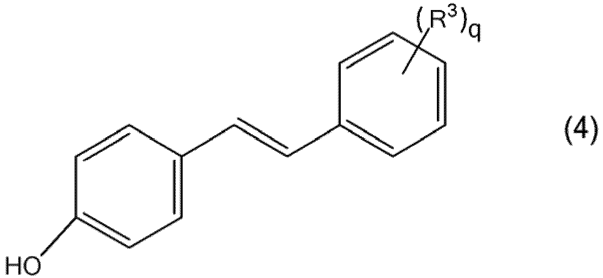 wherein R³ and q are as defined above.

The C₁₋₄ alkoxy group represented by R³ in formula (4) may be similar to that in formula (2). The position of the group R³ in formula (4) may be similar to that in formula (2).

The compound represented by formula (4) is a compound that can be produced by a known method from a known compound. The compound may also be obtained from commercial suppliers.

The compound represented by formula (5) is prepared as another starting material. 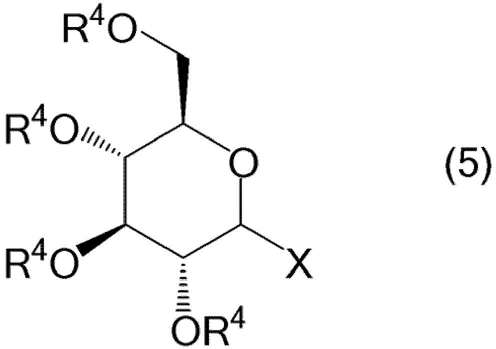 wherein X represents a halogen atom; and
R⁴ are the same or different, and each represents a protecting group of a hydroxyl group.

In formula (5), X represents a halogen atom. The halogen atom is not particularly limited. Examples of halogen atom include fluorine atom, chlorine atom, bromine atom, iodine atom, astatine atom, and the like. Among these halogen atoms, chlorine atom or bromine atom is preferable, and bromine atom is most preferable, in view of desorption capability from the compound represented by formula (5) and easy handling.

The linkage form of X with respect to the glucopyranose ring in formula (5) is not particularly limited. For example, the linkage may be an α-anomer or a β-anomer.

In formula (5), R⁴ represents a protecting group of a hydroxyl group. The protecting group of a hydroxyl group is not particularly limited. Examples include acetyl group, phosphate group, amino group, methyl group, benzyl group, p-methoxybenzyl group, tert-butyl group, methoxymethyl group, 2-tetrahydropyranyl group, ethoxyethyl group, benzoyl group, trimethylsilyl group, triethylsilyl group, tert-butyl dimethylsilyl group, triisopropylsilyl group, tert-butyl diphenylsilyl group, and the like. Among these protecting groups of a hydroxyl group, acetyl group, phosphate group, amino group, and methyl group are preferable, and acetyl group is particularly preferable.

In formula (5), R⁴ may be the same or different. In light of ease of production, all R⁴ are preferably the same. More specifically, it is most preferable that all of R⁴ in formula (5) are acetyl groups.

The compound represented by formula (5) is a compound that can be produced by a known method from a known compound. The compound may also be obtained from commercial suppliers.

By reacting the compounds represented by formulas (4) and (5) in the presence of a Lewis base, it is possible to produce a pterostilbene compound represented by formula (3).

The Lewis base is not particularly limited. Examples of the Lewis base include potassium carbonate, sodium carbonate, potassium hydrogen carbonate, sodium hydrogen carbonate, potassium hydroxide, sodium hydroxide, and the like. Among these, it is preferable to use potassium hydroxide. The Lewis bases listed above may be used solely, or in a combination of two or more.

In the method for producing the pterostilbene compound represented by formula (3), the solvent for reacting the mixture of the compounds represented by formulas (4) and (5) and the Lewis base is not particularly limited. Generally, C₁₋₄ lower alcohols may be used. Ethanol is preferable. In particular, anhydrous ethanol is preferable.

In the method for producing the pterostilbene compound represented by formula (3), the mixture of the compounds represented by formulas (4) and (5) and the Lewis base may be reacted under a condition with a predetermined temperature and a predetermined pressure. The predetermined temperature is not particularly limited. Generally, the reaction may be performed at a room temperature of about 10°C to 40°C. The predetermined pressure is not particularly limited. Generally, the reaction may be performed at an atmospheric pressure (about 1013 hPa).

In the method for producing the pterostilbene compound represented by formula (3), the mixture of the compounds represented by formulas (4) and (5) and the Lewis base may be reacted in the presence of a dehydration agent. By advancing the above reaction in the presence of a dehydration agent, the reaction time required in the method for producing the pterostilbene compound represented by formula (3) may be reduced. The dehydration agent is not particularly limited. Examples include aluminum oxide, calcium chloride, calcium oxide, anhydrous calcium sulfate, magnesium oxide, magnesium perchlorate, anhydrous magnesium sulfate, phosphorus oxide (V), silica gel, anhydrous sodium sulfate, sulfuric acid, zinc chloride, crystalline zeolite, and the like. Among these, crystalline zeolite is preferable; in particular, molecular sieve (3A, 4A, 5A, or 13X), which is a kind of crystalline zeolite, is more preferable.

To improve the purity of the pterostilbene compound represented by formula (3) obtained by the above method, it is also possible to use conventional isolation and/or purification means with respect to the mixture obtained after the reaction. Specific examples of isolation and/or purification means include chromatography, recrystallization, extraction, distillation, and the like.

### The case where p is an integer of 1 or more

It is possible to produce the pterostilbene compound represented by formula (2) wherein p is an integer of 1 or more by reacting the pterostilbene compound represented by formula (3) wherein p is 0 described above and a starch in the presence of cyclodextrin glycosyltransferase.

The starch is not particularly limited insofar as the starch is a compound having glucose molecules linked by a glycosidic linkage. Examples include soluble starch, dextrin, cyclodextrin, maltodextrin, and the like.

The cyclodextrin glycosyltransferase is an enzyme also known as a cyclodextrin glucanotransferase or CGTase. The cyclodextrin glycosyltransferase is not particularly limited insofar as it is an enzyme that catalyzes the reaction for adding other glucose molecules to the glucose group of a compound having a group derived from glucose molecule or glucose (glucose group). Examples include α-CGTase, β-CGTase, γ-CGTase, and the like. The material from which the cyclodextrin glycosyltransferase is obtained is also not particularly limited insofar as the function of catalyzing the above reaction can be exerted. Examples include microorganisms belonging to genus *Bacillus* and the like.

The reaction conditions in the case where p is an integer of 1 or more are not particularly limited insofar as the optimal conditions for the cyclodextrin glycosyltransferase to be used are ensured. For example, the reaction temperature is generally about 25 to 80°C. The reaction time is generally about 2 to 48 hours.

After the reaction is completed in the case where p is an integer of 1 or more, in order to improve the purity of the pterostilbene compound represented by formula (2), it is also possible to use conventional isolation and/or purification means with respect to the mixture obtained after the reaction. Specific examples of isolation and/or purification means include chromatography, recrystallization, extraction, distillation, and like means.

Further, after the reaction is completed, a known means may be used to isolate a specific compound, such as a pterostilbene compound wherein p is an integer of 2 or a pterostilbene compound wherein p is an integer of 3, among the pterostilbene compounds represented by formula (2). Generally, it is preferable to use chromatography.

### Examples

Examples are shown below to more specifically explain the present invention. However, the present invention is not limited to these Examples.

### Production Example 1

The pterostilbene monoglycoside represented by formula (6) was produced. 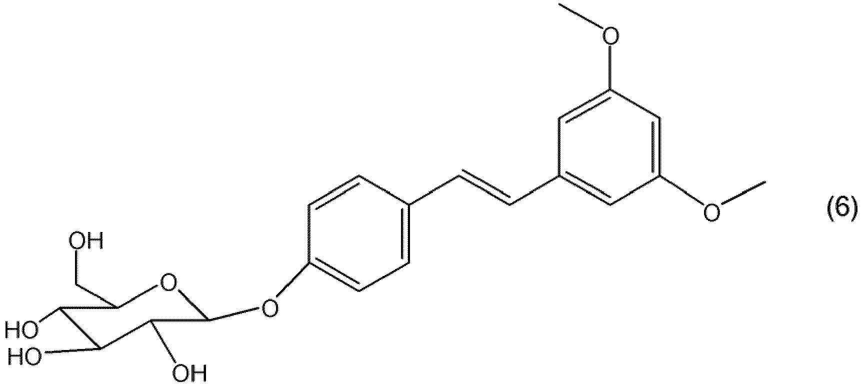 wherein the glycosidic linkage between the hydroxyl group at position 4 of pterostilbene and the hydroxyl group at position 1 of glucopyranoside is a β linkage.

The method for producing the pterostilbene monoglycoside described above is shown below. Pterostilbene, 2,3,4,6-tetra-O-acetyl-α-D-glucopyranosyl bromide (TAGluB), and potassium hydroxide at molar ratios of 1:1:2, respectively, were mixed with 2 mL of dry ethanol. The mixture was reacted at room temperature for 24 hours under a light-blocking condition with 5 mg of molecular sieve 4A. The product thus obtained from the reaction was dissolved in distilled water, and was subjected to fractionating extraction using ethyl acetate. The water-phase fraction thereof was further subjected to fractionating extraction using n-butanol.

The TAGluB described above was produced by the following method. 20 g of penta-O-acetyl-α-D-glucopyranose and 100 g of hydrogen bromide dissolved in acetic acid (25% solution) were weighed and stirred for 3 hours at room temperature under a light-blocking condition. The reaction product was subjected to extraction using ethyl acetate, and the extract was sequentially subjected to neutralization using a saturated sodium hydrogen carbonate aqueous solution, salting-out using saturated saline, and drying using magnesium sulfate, thereby producing TAGluB.

The physical property values of the pterostilbene production monoglycoside represented by formula (3) obtained by the production method described above are shown below.
¹H NMR(400 MHz in CD₃OD): 3.34-3.92(6H, multiplet, H-2"-H-6"), 3.78(6H, singlet, MeO, MeO), 4.92(1H, doublet, J=7.6 Hz, H-1"), 6.36 (1H, triplet, J=2.2 Hz, H-4), 6.67(2H, doublet, J=2.4 Hz, H-2, H-6), 6.96(1H, doublet, J=16.4 Hz, H-7), 7.08(1H, doublet, J=16.0 Hz, H-8), 7.08(2H, doublet, J=8.8 Hz, H-3',H-5'), 7.47(2H, doublet, J=8.8 Hz, H-2', H-6')

The physical property values of pterostilbene are as follows.
¹H NMR (400 MHz in CD₃OD): 3.78(6H, singlet, MeO, MeO), 6.34(1H, triplet, J=2.4 Hz, H-4), 6.65(2H, doublet, J=2.4 Hz, H-2, H-6), 6.77 (2H, doublet, J=8.4 Hz, H-3', H-5'), 6.88(1H, doublet, J=16.0 Hz, H-7), 6.95(1H, doublet, J=16.0 Hz, H-8), 7.37(2H, doublet, J=8.8 Hz, H-2', H-6').

### Production Example 2

### Pterostilbene Polyglycoside

The pterostilbene polyglycoside represented by formula (7) shown below was produced. 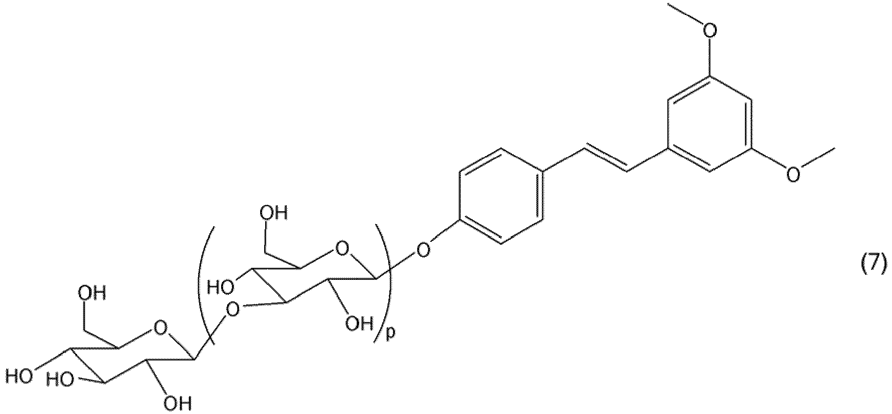 wherein n is an integer of 1 to 7; the glycosidic linkage of the oxygen atom positioned between the pterostilbene and the glucopyranoside ring with position 1" of the glucopyranoside ring is a β linkage; and all of the glycosidic linkages between the glucopyranoside rings are α(1-4) linkage.

The method for producing the pterostilbene polyglycoside described above is shown below. 1 g of the pterostilbene monoglycoside represented by formula (6) was dissolved in 40 mL of distilled water. 4 g of soluble starch (Nacalai Tesque, Inc.) was added to the solution, followed by incubation for 24 hours at 60°C in the presence of 0.5 m of cyclodextrin glycosyltransferase (Amano Enzyme Inc.). The enzyme is an enzyme that catalyzes a reaction to enable α(1-4) glycoside addition of other glucose molecules with respect to a compound having a glucose molecule.

The product thus obtained from the reaction was subjected to HPLC. Fig. 2A shows the results (HPLC charts). The HPLC conditions are as follows.
Column: YMC-Pack R&D ODS (φ20×150 mm)
Developing Solution: 35% Aqueous Solution of Acetonitrile
Flow Rate: 1 ml/min
Measurement Temperature: 40°C

A mixture of pterostilbene polyglycosides (about di- to hepta-) was obtained. Fig. 2A reveals that as the retention time in HPLC decreases, the number of glucose molecules linked to pterostilbene by an α(1-4) glycosidic linkage increases.

Further, by repeatedly performing HPLC under the above conditions with respect to the mixture, the pterostilbene diglycoside and pterostilbene diglycoside shown below were isolated.

Pterostilbene diglycoside is a compound represented by formula (8). 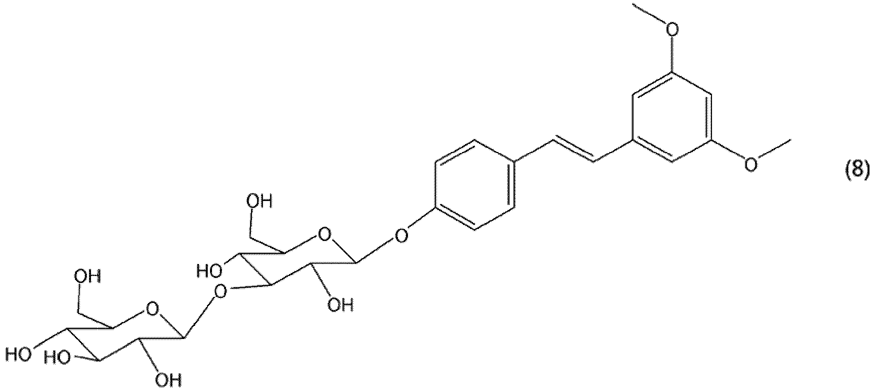 The linkage between position 4 of pterostilbene and glucose is β linkage, and the glycosidic linkage between glucose molecule is α(1-4) linkage. The physical property values (NMR and mass analysis) of the isolated pterostilbene diglycoside are as follows.

¹H NMR (400 MHz in CD₃OD): 3.29-3.92(12H, multiplet, H-2"-H-6", H-2‴-H-6‴), 3.79(6H, singlet, MeO, MeO), 4.95(1H, doublet, J=7.6 Hz, H-1"), 5.21(1H, doublet, J=3.6 Hz, H-1‴), 6.371H, triplet, J=2.0 Hz, H-4), 6.68(2H, doublet, J=2.4 Hz, H-2, H-6), 6.97(1H, doublet, J=16.8 Hz, H-7), 7.09(1H, doublet, J=16.8 Hz, H-8), 7.08(2H, doublet, J=8.8 Hz, H-3', H-5'), 7.47(2H, doublet, J=8.8 Hz, H-2', H-6')

Mass Analysis (ESI⁻): found: 579.2; estimate: 580.3

The pterostilbene triglycoside described above is a compound represented by formula (9).

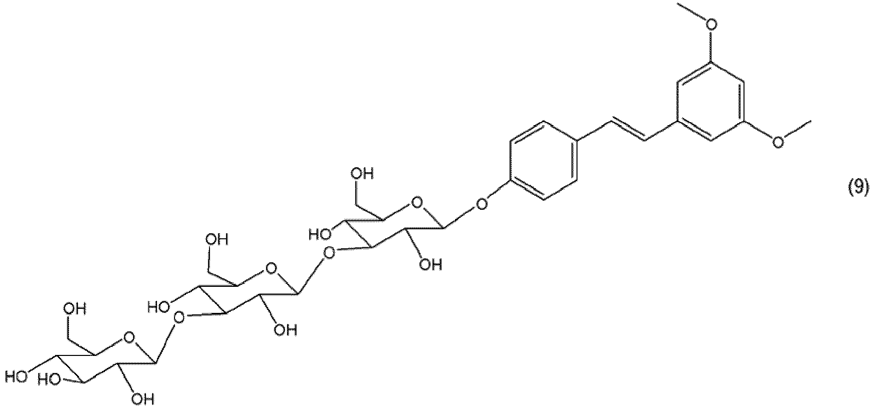

The linkage between position 4 of pterostilbene and glucose is β linkage, and the two glycosidic linkages between glucose molecules are both α(1-4) linkage. The physical property values (NMR and mass analysis) of the isolated pterostilbene diglycoside are as follows.

¹H NMR (400 MHz in CD₃OD): 3.29-3.89(18H, multiplet, H-2"-H-6", H-2‴-H-6‴, H-2ʺʺ-H-6ʺʺ), 3.79(6H, singlet, MeO, MeO), 4.95 (1H, doublet, J=8.0 Hz, H-1"), 5.16(1H, doublet, J=4.0 Hz, H-1ʺʺ), 5.21(1H, doublet, J=3.6 Hz, H-1‴), 6.37(1H, triplet, J=2.2 Hz, H-4), 6.68(2H, doublet, J=2.0 Hz, H-2, H-6), 6.97(1H, doublet, J=16.0 Hz, H-7), 7.09(1H, doublet, J=16.0 Hz, H-8), 7.08 (2H, doublet, J=8.8 Hz, H-3', H-5'), 7.48(2H, doublet, J=8.8 Hz, H-2', H-6')

Mass Analysis (ESI): found 741.2; estimate 742.3

### Example 1

### Water Solubility of Pterostilbene Glycoside

A test for confirming water solubility of the pterostilbene glycoside described above was performed. More specifically, solubility in 100 ml of distilled water at room temperature was measured. Pterostilbene (aglycone) was used as a control.

The results revealed that although the solubility was only about 5×10⁻⁴ g for aglycone, about 6×10⁻³ g for pterostilbene monoglycoside, and about 1.3×10⁻³ g for diglycoside (pterostilbene polyglycoside), the solubility was about 3.0×10¹ g for triglycoside.

The results revealed that the water solubility greatly differs between the diglycoside and triglycoside, which are both pterostilbene polyglycosides, and that triglycosides exert significantly superior water solubility.

### Example 2

### Test (1) for confirming production of Type-17 collagen protein in skin model cells

Pterostilbene (commercially available product of Tokyo Chemical Industry Co., Ltd.) and the pterostilbene monoglycoside represented by formula (3) above were added to a tissue produced by using EFT-400 human skin cell model (Kurabo Industries Ltd.) at final concentrations of 25 µM and 50 µM, respectively, and the tissue was cultured for 7 days. A tissue containing DMSO was prepared as a control.

The lysates of cultured tissue were subjected to western blotting using an anti-Type-17 collagen antibody (ab95123, Abcam). Fig. 1 shows the results. Graph A in the figure shows the results of western blotting showing the amount of Type-17 collagen protein in a human skin model in which pterostilbene was added, and graph B shows the results of addition of pterostilbene monoglycoside.

Fig. 1A revealed that pterostilbene increased the amount of Type-17 collagen contained in human skin model cells.

More specifically, in comparison with the control, the expression amount of Type-17 collagen increased by about 1.1 times by the addition of 25 µM, and increased by about 1.4 times by the addition of 50 µM (western blotting was performed using α-GAPDH antibody as the internal standard; data are not shown).

Further, Fig. 1B revealed that pterostilbene monoglycoside increased the amount of Type-17 collagen contained in human skin model cells. Further, in comparison with the control, the amount of Type-17 collagen increased by about 1.4 times by the addition of 25 µM, and increased by about 1.6 times by the addition of 50 µM (western blotting was performed using α-GAPDH antibody as the internal standard; data are not shown).

### Example 3

### Test (2) for confirming production of Type-17 collagen protein in skin model cells

Western blotting was performed for confirming the production amount of Type-17 collagen protein in skin cell model using the pterostilbene diglycoside and the pterostilbene triglycoside produced in Production Example 2 described above.

The skin cell model used, the anti-Type-17 collagen antibody, the α-GAPDH antibody as the internal standard, and the test method were similar to those used in Example 2 described above.

Fig. 2 revealed that pterostilbene diglycoside increased the amount of Type-17 collagen contained in skin model cells by 1.2 times, compared with the control (Ctrl). It was further revealed that pterostilbene triglycoside increased the amount of Type-17 collagen by 1.6 times, compared with the control.

The results of these Test Examples 1 and 2 revealed that, in addition to pterostilbene, pterostilbene glycosides (monoglycoside and polyglycoside) as the polyglycosides of stilbene compounds are also stilbene compounds capable of exerting the effects of increasing the production amount of Type-17 collagen production amount in a human skin model.

### Formulation Examples

Various cosmetic compositions are produced in accordance with the formulations shown in Tables 1 to 4 below.

**[Table 1]**

| Formulation Example 1: Skin Cream (40 g) | |
|---|---|
| Scualane | 15 wt% |
| Glycerin | 10 wt% |
| Betaine | 2 wt% |
| Cyclomethicone | 1.5 wt% |
| Trehalose | 1 wt% |
| BG | 4 wt% |
| Dimethicone | 1 wt% |
| 1,2-Hexanediol | 0.5 wt% |
| Caprylyl Glycol | 0.5 wt% |
| Stilbene Compound and | 0.5 wt% |
| Glycoside Thereof | |
| Ubiquinone | 0.03 wt% |
| Orange Flower Extract | 0.1 wt% |
| Glucosamine Hydrochloride | 0.2 wt% |
| PEG-20 Sorbitan Cocoate | 1 wt% |
| Dipotassium Glycyrrhizate | 0.05 wt% |
| Dextrin | 0.07 wt% |
| Carbomer | 0.9 wt% |
| Sodium Carbonate | 0.45 wt% |
| Water | Balance |
| | 100 wt% |

**[Table 2]**

| Formulation Example 2: Lotion (120 ml) | |
|---|---|
| Glycerin | 10 wt% |
| Trehalose | 2 wt% |
| 1,2-Hexanediol | 0.5 wt% |
| Caprylyl Glycol | 0.5 wt% |
| BG | 5 wt% |
| Stilbene Compound and | 0.5 wt% |
| Glycoside Thereof | |
| Hydrolyzed Arginine | 0.5 wt% |
| Hydrolyzed Collagen | 0.1 wt% |
| Orange Flower Extract | 0.1 wt% |
| Arginine Ferulate | 0.1 wt% |
| Ubiquinone | 0.01 wt% |
| Dextrin | 0.02 wt% |
| PEG-20 Sorbitan Cocoate | 0.5 wt% |
| Dipotassium Glycyrrhizate | 0.05 wt% |
| Carbomer | 0.2 wt% |
| Sodium Carbonate | 0.1 wt% |
| Water | Balance |
| | 100 wt% |

**[Table 3]**

| Formulation Example 3: Shampoo (300 ml) | |
|---|---|
| Sodium Cocoyl Glutamate 25% Aqueous | 25 wt% |
| Solution | |
| Sodium Methyl Cocoyl Taurate 30% | 10 wt% |
| Aqueous Solution | |
| Lauryl Betaine | 16 wt% |
| Cocamide DEA | 1 wt% |
| Betaine | 1 wt% |
| Polyquaternium-10 | 0.8 wt% |
| Stilbene Compound and Glycoside | 0.5 wt% |
| Thereof | |
| EDTA-2Na | 0.1 wt% |
| Citric Acid | 0.4 wt% |
| Methylparaben | 0.2 wt% |
| Purified Water | Balance |
| | 100 wt% |

**[Table 4]**

| Formulation Example 4: Body Soap (300 ml) | |
|---|---|
| Sekken Soji-K (JPN) 30% Aqueous | 50 wt% |
| Solution | |
| Lauramidopropyl Betaine 30% Aqueous | 15 wt% |
| Solution | |
| Sodium Laureth Sulfate 25% Aqueous | 15 wt% |
| Solution | |
| Glycol Distearate | 1.5 wt% |
| 1,3-Butylene Glycol | 4 wt% |
| Stilbene Compound and Glycoside | 0.5 wt% |
| Thereof | |
| Citric Acid | Appropriate wt% |
| Hydroxypropyl Methylcellulose | 0.3 wt% |
| Purified Water | Balance |
| | 100 wt% |

Various food or beverage compositions are produced in accordance with the formulations shown in Tables 5 and 6 below.

**[Table 5]**

| Formulation Example 5: Tablet | |
|---|---|
| Dietary Fiber (Inulin) | 62.5 mg |
| Beer Yeast | 26.5 mg |
| Coenzyme Q₁₀ | 22.5 mg |
| Stilbene Compound and Glycoside | 5 mg |
| Thereof | |
| Curcuma Zedoaria Powder | 18 mg |
| Autumn Turmeric | 13 mg |
| Vitamin Pre-Mix | 38 mg |
| Other Excipients | 50 mg |
| | 100 wt% |

Vitamin Premix includes Vitamin C, Vitamin A, Vitamin B₁, Vitamin B₂, Vitamin B₆, Vitamin B₁₂, and Pantothenic Acid; and is available from PC-System Inc. or the like.

**[Table 6]**

| Formulation Example 6: Drink (50 ml) | |
|---|---|
| Fish Collagen | 12 wt% |
| Red Grape Juice | 5 wt% |
| Stilbene Compound and | 0.5 wt% |
| Glycoside Thereof | |
| Hyaluronic Acid | 0.04 wt% |
| GABA | 0.05 wt% |
| L-Ornithine | 0.1 wt% |
| Vitamin P | 0.1 wt% |
| Vitamin Premix | 0.1 wt% |
| Galactooligosaccharide | 4 wt% |
| Erythritol | 4 wt% |
| Flavor (Grape) | 0.2 wt% |
| Water | Balance |
| | 100 wt% |

Vitamin Premix includes Vitamin C, Vitamin B₁, Vitamin B₂, Vitamin B₆, Vitamin B₁₂, and Sodium Citrate; and is available from PC-System Inc. or the like.

## Claims

1. A non-therapeutic cosmetic use for promoting Type-17 collagen production of a cosmetic composition comprising pterostilbene or a glycoside thereof as an active ingredient.

2. The use according to claim 1, wherein the glycoside is a glycoside in which pterostilbene and the hydroxyl group at position 1 of a saccharide are linked.

3. The use according to claims 1 or 2, wherein the glycoside is a glycoside in which pterostilbene and a monosaccharide are linked.

4. The use according to claim 3, wherein the monosaccharide is at least one member selected from the group consisting of glucose, threose, lyxose, allose, psicose, sedoheptulose, and volemitol.

5. The use according to claims 1 or 2, wherein the glycoside is a glycoside in which pterostilbene and a polysaccharide constituted of two or more saccharides are linked.

6. The use according to claim 5, wherein the saccharide residue of the polysaccharide linked to the pterostilbene is a glucose residue.

## Patentansprüche

1. Nicht therapeutische kosmetische Verwendung zum Fördern einer Typ-17-Kollagenproduktion einer kosmetischen Zusammensetzung, umfassend Pterostilben oder ein Glykosid davon als ein Wirkstoff.

2. Verwendung nach Anspruch 1, wobei das Glykosid ein Glykosid ist, in dem Pterostilben und die Hydroxylgruppe an Position 1 eines Saccharids verknüpft sind.

3. Verwendung nach Anspruch 1 oder 2, wobei das Glykosid ein Glykosid ist, in dem Pterostilben und ein Monosaccharid verknüpft sind.

4. Verwendung nach Anspruch 3, wobei das Monosaccharid mindestens ein Element ist, das aus der Gruppe ausgewählt ist, bestehend aus Glukose, Threose, Lyxose, Allose, Psicose, Sedoheptulose und Volemitol.

5. Verwendung nach Anspruch 1 oder 2, wobei das Glykosid ein Glykosid ist, in dem Pterostilben und ein Polysaccharid, das aus zwei oder mehr Sacchariden besteht, verknüpft sind.

6. Verwendung nach Anspruch 5, wobei der Saccharidrückstand des Polysaccharids, das mit dem Pterostilben verknüpft ist, ein Glukoserückstand ist.

## Revendications

1. Utilisation cosmétique non thérapeutique destinée à favoriser la production de collagène de type 17 d'une composition cosmétique comprenant du ptérostilbène ou un glycoside de celui-ci comme ingrédient actif.

2. Utilisation selon la revendication 1, dans laquelle le glycoside est un glycoside dans lequel le ptérostilbène et le groupe hydroxyle en position 1 d'un saccharide sont liés.

3. Utilisation selon les revendications 1 ou 2, dans laquelle le glycoside est un glycoside dans lequel le ptérostilbène et un monosaccharide sont liés.

4. Utilisation selon la revendication 3, dans laquelle le monosaccharide est au moins un élément choisi dans le groupe constitué par le glucose, le thréose, le lyxose, l'allose, le psicose, le sédoheptulose et le volémitol.

5. Utilisation selon les revendications 1 ou 2, dans laquelle le glycoside est un glycoside dans lequel le ptérostilbène et un polysaccharide constitué de deux saccharides ou plus sont liés.

6. Utilisation selon la revendication 5, dans laquelle le résidu saccharide du polysaccharide lié au ptérostilbène est un résidu glucose.
